# EUROPÄISCHE PATENTSCHRIFT

(11) **EP 0 158 986 B1**
(45) Veröffentlichungstag und Bekanntmachung des Hinweises auf die Patenterteilung: **15.09.1993**
(21) Anmeldenummer: 85104445.3
(22) Anmeldetag: 12.04.1985
(51) Int. Cl.: C07K 7/10, A61K 37/02

(54) **Neue Polypeptide mit blutgerinnungshemmender Wirkung, Verfahren zu deren Herstellung bzw. Gewinnung, deren Verwendung und diese enthaltende Mittel**
Peptides having an anticoagulant activity, process for their preparation, their isolation and use, and agents containing them
Peptides à activité anticoagulante, leur procédé de préparation, leur isolement, leur emploi et les agents les contenant

(30) Priorität: 18.04.1984 DE 3414593; 19.10.1984 DE 3438296
(43) Veröffentlichungstag der Anmeldung: 23.10.1985
(73) Patentinhaber: HOECHST AKTIENGESELLSCHAFT, 65926 Frankfurt am Main (DE)
(72) Erfinder: Tripier, Dominique, Dr., D-6239 Eppstein/Taunus (DE)

(56) Entgegenhaltungen:
- EP-A- 0 142 860
- EP-A- 0 158 564
- EP-A- 0 171 024
- DIE PHARMAZIE, Band 36, Nr. 10, Oktober 1981, Seiten 653-660, VEB Verlag Volk und Gesundheit, Berlin, DE; P. WALSMANN et al.: "Biochemische und pharmakologische Aspekte des Thrombininhibitors Hirudin"
- Protides Biol. Fluids, Proc. Coll. 1975, vol.23, S. 307-313

## Beschreibung

Antikoagulantien dienen der Prophylaxe und Therapie thrombo-embolischer Prozesse; ihr Haupteinsatzgebiet liegt dabei vor allem bei venösen Thromboembolien. Antikoagulantien werden ferner bei der Herstellung von Blutkonserven benötigt. Derivate des 4-Hydroxycumarins oder des 1,4-Indandions, die beispielsweise für diesen Zweck angewendet werden, weisen trotz weitgehender Optimierung eine Reihe von Nachteilen auf (vgl. z.B. Mutschler, Arzneimittelwirkungen, 4. Auflage, Stuttgart 1981, Seite 375 ff.).

Es ist daher wünschenswert, besonders in der Humanmedizin Blutgerinnungshemmer zur Verfügung zu haben, die eine geringe Toxizität und wenig Nebenwirkungen aufweisen und die durch ihren Metabolismus keine Belastung des erkrankten Organismus darstellen.

Außer den körpereigenen plasmatischen Hemmstoffen wie Antithrombin III besitzen auch viele andere Proteine blutgerinnungshemmende Wirkung, wie z.B. der Kunitz-Inhibitor, der aus Sojabohnen gewonnen wird. Dieser Hemmstoff blockt die Blutgerinnungskaskade durch Inhibition des aktivierten Faktors Xa, aber die Spezifität des Inhibitors ist so gering, daß viele Nebenwirkungen entstehen: Hemmung des Plasmakallikreins, des Plasmins, des Trypsins, so daß die therapeutischen Anwendungen ausgeschlossen sind. Auch andere Wirkstoffe, wie der Ascaris- oder der Kazals-Inhibitor, konnten wegen mangelnder Spezifität keine Bedeutung erlangen.

Hirudin (The Merck Index, 9. Auflage, Rahway 1976, Seite 618; Pharmazie 36 [1981] Heft 10), ein aus Hirudo medicinalis gewonnenes Polypeptid, zeigt dagegen eine spezifische Antithrombin-Aktivität (vgl. z.B. Markwardt, Blutgerinnungshemmende Wirkstoffe aus blutsaugenden Tieren, Jena 1963). Das aufwendige Verfahren zu seiner Isolierung und Reinigung hat sich bisher nachteilig auf seine praktische Anwendung ausgewirkt.

Es wurde nun überraschenderweise gefunden, daß sich aus Blutegeln ein hochaktives Polypeptid der Formel II, in der R Wasserstoff oder SO₃H bedeutet, isolieren läßt. 
1 2 3 4 5 6 7 8 9 10 11 12 13 14
H-Thr-Tyr-Thr-Asp-Cys-Thr-Glu-Ser-Gly-Gln-Asn-Leu-Cys-Leu-
15 16 17 18 19 20 21 22 23 24 25 26 27 28
-Cys-Glu-Gly-Ser-Asn-Val-Cys-Gly-Gln-Gly-Asn-Lys-Cys-Ile-
29 30 31 32 33 34 35 36 37 38 39 40 41 42
-Leu-Gly-Ser-Asp-Gly-Glu-Lys-Asn-Gln-Cys-Val-Thr-Gly-Glu-
43 44 45 46 47 48 49 50 51 52 53 54 55 56
-Gly-Thr-Pro-Lys-Pro-Gln--Ser-His-Asn-Asp-Gly-Asp-Phe-Glu-
57 58 59 60 61 62 63 64
-Glu-Ile-Pro-Glu-Glu-Tyr(R)-Leu-Gln-OH

(II)
Die Erfindung betrifft daher Polypeptide der Formel I 
1 2 3 4 5 6 7 8 9 10 11 12 13 14
H- -Y-Tyr-Thr-Asp-Cys-Thr-Glu-Ser-Gly-Gln-Asn-Leu-Cys-Leu-
15 16 17 18 19 20 21 22 23 24 25 26 27 28
-Cys-Glu-Gly-Ser-Asn-Val-Cys-Gly-Gln-Gly-Asn-Lys-Cys-Ile-
29 30 31 32 33 34 35 36 37 38 39 40 41 42
-Leu-Gly-Ser-Asp-Gly-Glu-Lys-Asn-Gln-Cys-Val-Thr-Gly-Glu-
43 44 45 46 47 48 49 50 51 52 53 54 55 56
-Gly-Thr-Pro-Lys-Pro-Gln-Ser-His-Asn-Asp-Gly-Asp-Phe-Glu-
57 58 59 60 61 62 63 64
-Glu-Ile-Pro-Glu-Glu-Tyr(R)-Leu-Gln--OH

(I)
in welcher
- R: phenolischer Wasserstoff oder SO₃H bedeutet,
- Y: Val, Ile, Thr, Leu oder Phe bedeutet und
in der jeweils 2 der 6 Cys-Reste in den Positionen 5, 13, 15, 21, 27 und 38 über Disulfid-Brücken verknüpft sind,
sowie deren physiologisch verträglichen Salze.

Als Salze kommen insbesondere Alkali- und Erdalkalisalze, Salze mit physiologisch verträglichen Aminen sowie Salze mit physiologisch verträglichen Säuren, wie HCl, H₂SO₄, Maleinsäure oder Essigsäure in Frage.

Die Erfindung betrifft auch biologisch aktive peptidische Spaltprodukte, die durch chemische oder enzymatische Spaltung dieser Polypeptide erhältlich sind.

Die Erfindung betrifft weiterhin ein Verfahren zur Gewinnung eines gereinigten Polypeptids der obengenannten Formel, das dadurch gekennzeichnet ist, daß man das Polypeptids aus Würmern des Stammes Annelida mit Hilfe einer Kombination von Extraktionsmethoden, Fällungsmethoden, Membranfiltration und/oder chromatographischen Verfahren isoliert und das erhaltene Peptid gegebenenfalls in seine physiologisch verträglichen Salze überführt.

Das Polypeptid wird vorzugsweise aus den Halsdrüsen von Würmern der Klasse Hirudinea, insbesondere aus solchen der Ordnung Gnathobdellida gewonnen. Bevorzugt sind die Gattungen Hirudo und Haemodipsa. Besonders bevorzugt ist die Gattung Hirudo, daraus insbesondere Hirudo medicinalis. Neben den Halsdrüsen des Blutegels kann auch dessen vordere Körperregion oder der ganze Blutegel Verwendung finden.

Ein Verfahren zur Gewinnung eines Rohextraktes aus Blutegeln ist in Enzymoly, Band 5 "Hirudin as an Inhibitor of Thrombin" beschrieben. Ein Reinigungsverfahren für Hirudin ist aus Bull. Soc. Chim. Biol. 45 [1963] 55 bekannt.

Beim erfindungsgemäßen Verfahren hat sich insbesondere eine Kombination von Fällungsmethoden und von Gelpermeations-Chromatographie oder Ultrafiltration und von hoch auflösender Verteilungschromatographie an über "Reverse-Phase"-Material und Chromatographie an Kieselgel oder Aluminumoxid als nützlich erwiesen. Je nach Beschaffenheit des Rohextraktes können aber auch andere Chromatographie-Verfahren vorteilhaft angesetzt werden (evtl. auch in Kombination mit dem obengenannten Verfahren), wie z.B. Kationen- oder Anionen-Austausch-Chromatographie, Chromatographie an unspezifischen Absorbentien, insbesondere Hydroxylapatit.

Um beispielsweise einen für die Chromatographie geeigneten Rohextrakt zu gewinnen, können die Kopfteile des Blutegels in gefrorenem Zustand zerkleinert und mittels einer wäßrigen Puffer-Lösung (z.B. Phosphatpuffer) extrahiert werden. Das unlösliche Material wird z.B. durch kurzes Zentrifugieren oder durch Filtration über Gaze abgetrennt und das Polypeptid aus dem so erhaltenen Extrakt abgetrennt und isoliert. Es ist vorteilhaft, diesen Extrakt schnell auf 70° bis 90°C zu erhitzen, weil dadurch die Hauptmenge der proteolytischen Enzyme denaturieren und ausfallen, deren Abtrennung dann z.B. durch Zentrifugation erfolgen kann. Man isoliert aus dem Extrakt die Proteinfraktion, die das erfindungsgemäße Peptid enthält, z.B. durch Fällung in der Weise, daß man den Extrakt in ein wassermischbares organisches Lösungsmittel gibt. Z.B. kann Aceton in einer mehrfachen Menge des Extraktvolumen, vorzugsweise der etwa 10-fachen Menge eingesetzt werden, wobei die Fällung in der Kälte, üblicherweise bei 0 bis -40°C, vorzugsweise bei etwa -20°C, vorgenommen wird.

Aus diesem Rohextrakt können Proteine mit höherem Molekulargewicht z.b. durch Ultrafiltration oder durch Gelpermeabilitäts-Chromatographie abgetrennt werden. Bei größeren Ansätzen kann die Ultrafiltation z.B. in zwei Stufen erfolgen: In der ersten Stufe arbeitet man mit einer Kapillarmembran mit einer Ausschlußgrenze von 500 000 Dalton und dann in der zweiten Stufe mit einer Flachmembran mit einer Ausschlußgrenze von 10 000 Dalton. Mit Hilfe der Kapillarmembran erreicht man eine schnelle Abtrennung von höhermolekularem Material, welches den Durchfluß der selektiven arbeitenden Flachmembran verhindern würde. Bei kleinen Mengen kann man auch auf die erste Stufe der Ultrafiltation verzichten.

Eine andere Möglichkeit, die Fällung durchzuführen, ist die Zugabe von Salzen, wie z.B. Ammoniumsulfat. Durch pH-Steuerung erreicht die Fällung eine gewisse Selektivität. Das erfindungsgemäße Peptid, das einen isoelektrischen Punkt von 3,9 hat, kann im pH-Bereich zwischen 3 und 5, vorzugsweise etwa 4, durch Zugabe von Ammoniumsulfat bis zu einer Konzentration von etwa 50 % ausgefällt werden, wobei eine Vielzahl von Begleitproteinen dabei in der Lösung bleibt. Auch diese Fällung wird unter Kühlung bei etwa -5 bis +15°C vorzugsweise zwischen 0 und +4°C durchgeführt.

Das so erhaltene Material besteht aufgrund der bisher angewandten Methode immer noch aus einer Mischung von Polypeptiden. Ein bevorzugtes Verfahren zur Gewinnung des Inhibitors der Formel II mit R= H bzw. SO₃H besteht darin, daß der Rohextrakt durch ein, oder mehrere, hochauflösende Chromatographie-Systeme fraktioniert wird. Diese Fraktion kann wiederum durch ein zweites hochauflösendes chromatographisches System in einzelnen Komponenten aufgelöst werden, um somit die Inhibitor der Formel II zu isolieren.

Als erste chromatographische Stufe bei der Gewinnung dieser Inhibitoren haben sich Verfahren, wie sie z.B. aus der EP-A-82359 bekannt sind, als vorteilhaft erwiesen. dabei trennt man die Proteingemische auf herkömmlichem Kieselgel mit geeigneter Korngröße oder auf mit Kieselgel-Fertigsäulen (wie z.B. die Lobar^{R}-Säule) mit Hilfe eines Puffersystems.

Die Anbringung der Probe auf die Säule kann auf einer voräquilibrierten Säule durchgeführt weiden. Ohne Nachteil kann man das Substanzgemisch aber auch auf die trockene Säule anbringen.

Ein Verhältnis zwischen dem Proteingemisch und den Absorbentien zwischen 1 : 50 bis 1 : 200 hat sich als vorteilhaft erwiesen.

Die Elution kann mit einem Puffer aus Chloroform, Methanol, Eisessig, Wasser und Triethylamin erfolgen. Man kann auch andere Pufferlösungen dazu verwenden, wie z.B. 70 % Ethanol, 30 % Tris-Puffer (0,05 M, pH 8,0).

Der letzte Reinigungsschritt besteht aus einer chromatographischen Trennung auf "Reverse-Phase"-Material. Durch das hohe Auflösungsvermögen der HPLC-Technologie vgl. z.B. "High Performance Liquid Chromatography Advances and Perspectives", Band 3, Csaba Horvàth, Academic Press, 1983, Seite 50 - 83 oder in "Methods of Enzymology", Band 91, Seite 137 - 190 und 352 - 359, 1983 ist es möglich, die Inhibitoren der Formel II von Begleitproteinen zu trennen und rein darzustellen.

Für die stationäre Phase haben sich derivatisierte Kieselgele mit geeigneter Korngröße (z.B. zwischen 3 und 20 µm) vorteilhaft erwiesen. Für die Derivatisierung des Kieselgels eignen sich neben den verbreiteten Octadecylsilanresten auch eine Vielzahl anderer Silanreste oder deren Mischungen, wie Silanreste mit niedrigen Alkyl, Phenylalkyl- oder amino-substituiertem Alkyl, wobei letztere eine gewisse Kombination von Ionenaustausch- und "Reverse-Phase"-Chromatographie bieten. Es können beispielsweise Trennsäulen von 5 bis 25 cm Länge und einen Durchmesser von 3 bis 10 mm verwendet werden. Als gepuffertes Elutionsmittel kommen alle sekundären oder tertiären Mischungen zwischen Wasser, organischen Lösungsmitteln geeigneter Lipophilie in Frage, wie z.B. niedrige Alkohole, Ketone, Nitrile, Ether, Säure, Amine, Glykolether, Amide und deren Derivate. Als Puffersubstanz können organische und anorganische Salze oder andersartige Zusätze verwendet werden. Die Elution erfolgt vorteilhaft bei einem pH-Wert zwischen 2 und 8.

Die Benutzung von flüchtigen Puffersubstanzen, wie Ammoniumacetat oder Ammoniumbicarbonat, erlaubt die Gewinnung der Inhibitoren aus dem Eluat durch einfache Gefriertrocknung.

Das erfindungsgemäße Polypeptid der Formel II ist farblos, im Wasser und in wäßrigen Puffern löslich, zeigt sich homogen in der Polyacrylamid-Elektrophorese und besitzt einen isoelektrischen Punkt von 3,9 (bestimmt durch isoelektrische Fokussierung). Bestimmt man die Aminosäurezusammensetzung nach der Methode von Moore und Stein (Methods of Enzymology Band VI, 819 - 831, herausgegeben von Rolovick und Kaplan, Academic Press, New York, London, 1963), findet man folgende Werte:
9 Asparaginsäure, 5 Threonin, 4 Serin, 13 Glutaminsäure, 3 Prolin, 9 Glycin, 2 Valin, 6 Cystein, 2 Isoleucin, 4 Leucin, 2 Tyrosin, 1 Phenylalanin, 3 Lysin und 1 Histidin.

Die Erfindung betrifft ferner ein Verfahren zur Herstellung eines Polypeptids der obengenannten Formel I, das dadurch gekennzeichnet ist, daß man
a) es in an sich bekannter Weise mittels Festphasensynthese herstellt oder
b) zur Herstellung eines Polypeptids, in welchem n= 0 ist,
   I. Hirudin einem zweifachen Edman-Abbau unterwirft,
   II. das so erhalten Peptid mit einem Aktivester einer Aminosäure oder eines Peptids der Formel U-(X)ₘ-Y-OH, in welcher m, X und Y wie oben definiert sind und U für eine säuren- oder basenlabile Urethanschutzgruppe steht, umsetzt,
   III. die Phenylthiocarbamoyl-Gruppe an der ε-Amino-Funktion von Lys mittels Hydrazin
   IV. und die Urethan-Schutzgruppe U mit Hilfe einer Säure oder Base abspaltet und das a) oder b) erhaltene Polypeptid gegebenenfalls in sein physiologisch vorträgliches Salz überführt.

Bei der Festphasensynthese (vgl. hierzu Atherton, Sheppard, Perspectives in Peptide Chemistry, Karger Basel 1981, Seiten 101 - 117) kann in der Regel auf eine OH-Schutzgruppe für Thr verzichtet werden.

Die Synthese des Polypeptids der Formel I erfolgt z.B. schrittweise an hydroxymethyliertem Polystyrol-Harz. Das Polystyrol ist mit beispielsweise 1 % Divinylbenzol quervernetzt. Es liegt gewöhnlich in Form kleiner Kügelchen vor.

Die Aminosäuren werden N-terminal geschützt eingesetzt. Die erste N-geschützte Aminosäure wird per Esterbildung am Träger angebracht. Nach Beseitigung der Aminoschutzgruppe wird die nächste N-geschützte Aminosäure unter Verwendung eines Kupplungsreagenzes wie Dicyclohexylcarbodiimid angeknüpft. Entschützen und Zufügen weiterer Aminosäuren wird fortgesetzt, bis die gewünschte Sequenz erreicht ist.

Die Auswahl der Schutzgruppen richtet sich nach den Aminosäuren und Kupplungsmethoden.

Als Aminoschutzgruppen kommen z.B. die bekannten urethanischen Schutzgruppen wie Benzyloxycarbonyl(Z), p-Methoxycarbobenzoxy, p-Nitrocarbobenzoxy, t-Butyloxycarbonyl(Boc), Fmoc und ähnliche in Frage.

Die Boc-Gruppe wird bevorzugt, da sie durch relativ milde Säuren (z.B. Trifluoressigsäure oder HCl in organischen Lösungsmitteln) abspaltbar ist.

Threonin kann als Benzylether blockiert werden und die ε-Aminofunktion des Lysins als Z-Derivat. Diese beiden Schutzgruppen sind gegen die Abspaltungsreagenzien für die Boc-Gruppe weitestgehend resistent und können hydrogenolytisch mit einem Hydrierkatalysator (Pd/Aktivkohle) oder z.B. mit Natrium in flüssigem Ammoniak entfernt werden.

Das geschützte Peptid kann z.B. mit Hydrazin vom Harz genommen werden. Dabei entsteht das Hydrazid, welches z.B. mit N-Bromsuccinimid nach Int. J. Pept. Prot. Research 17 [1981] 6 - 11 in die freie Carbonsäure überführt werden kann. Falls erforderlich, müssen die Disulfid-Brücken oxidativ geschlossen werden (vgl. König, Geiger, Perspectives in Peptide Chemistry, Karger Basel Seiten 31 - 44).

Bei der Verfahrensvariante b) unterwirft man Hirudin einem zweifachen Edman-Abbau, indem man dieses Polypeptid in einer geeigneten Pufferlösung, wie Pyridin/Wasser oder Dioxan/Wasser gegebenenfalls unter Zusatz einer Base wie NaOH oder Triethylamin, vorzugsweise bei etwa 40°C und einem pH-Wert von 8 - 9 fit einem Isothiocyanat, vorzugsweise Phenylisothiocyanat umsetzt. Durch Behandeln mit einer Säure (z.B. 3N HCl bei Raumtemperatur und anschließendes Erwärmen auf 40°C) wird das N-terminale Valin als Phenylthiazolinon abgespalten. Man wiederholt diese Reaktionsfolge zur Spaltung des zweiten Valins am N-Terminus.

Das auf diese Weise erhaltene Des-(Val)₂-Hirudin-Derivat wird mit einem Aktivester einer Aminosäure oder eines Paptids der Formel U-(X)ₘ-Y-OH umgesetzt. Geeignet sind z.B. p-Nitrophenyl-, Cyanomethyl-, N-Hydroxyphthalimid- oder insbesondere N-Hydroxysuccinimid-Ester. Geeignete Urethanschutzgruppen U sind solche, die sauer oder alkalisch abspaltbar sind, wie z.B. Boc oder Msc. Falls erforderlich, so können auch evtl. vorhandene Funktionen in den Seitenketten von X und Y durch geeignete Schutzgruppen vorübergehend geschützt werden.

Die auf diese Weise erhaltene geschützte Vorstufe des Polypeptids der Formel I (n=0) wird zur Abspaltung der Phenylthiocarbamoyl-Gruppe am Lysin in einem geeignetem Lösungsmittel, wie einem niedrigen Alkohol oder dessen Gemisch mit Wasser mit Hydrazin-Hydrat behandelt.

Man spaltet aus diesem Polypeptid nun noch die restliche(n) Schutzgruppe(n) in geeigneter Weise ab (Boc z.B. mit Trifluoressigsäure, MSC mit einer Base) und erhält so das erfindungsgemäße Polypeptid der Formel I.

Die erfindungsgemäßen Polypeptide sind spezifische stöchiometrische Hemmer des Thrombins. Die quantitative Messung der Thrombin-Hemmung durch den erfindungsgemäßen Inhibitoren zeigte, daß der Komplex Inhibitor-Thrombin praktisch nicht dissoziiert. Mit Hilfe dieser Meßmethode kann während der Aufarbeitung und Reinigung die Aktivität und damit der Reinheitsgrad der erfindungsgemäßen Polypeptide bestimmt werden. Das so gereinigte Polypeptid der obengenannten Formel II kann dabei eine Thrombinhemmung von über 10 000 ATU/mg aufweisen und damit die des herkömmlichen Hirudins übertreffen.

Die Erfindung betrifft daher auch die Verwendung von Polypeptiden der Formel I, in welcher R, und Y die obengenannte Bedeutung haben und Y darüberhinaus auch eine chemische Bindung bedeuten kann, als Blutgerinnungshemmer zur Anwendung bei der Therapie thromboembolischer Prozesse sowie deren Anwendung als Diagnostika und Reagentien.

Die Erfindung betrifft weiterhin Mittel, die ein Polypeptid der Formel I in einem pharmazeutisch unbedenklichen Träger enthalten und Verfahren zu deren Herstellung, dadurch gekennzeichnet, daß man diese in eine geeignete Darreichungsform bringt.

Die erfindungsgemäßen Verbindungen können parenteral oder topisch in entsprechender pharmazeutischer Zubereitung verabreicht werden.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren, Isotoniemittel, Konservierungsstoffe oder weitere Hilfsstoffe in Lösung, Suspension oder Emulsion gebracht. Als Lösungsmittel für die neuen aktiven Verbindungen und die entsprechenden physiologisch verträglichen Salze kommen z.B. in Frage: Wasser, physiologische Kochsalzlösungen oder Alkohole, z.B. Ethanol, Propandiol, oder Glycerin, daneben auch Zuckerlösungen wie Glucose- oder Mannitlösungen, oder auch eine Mischung aus den verschiedenen genannten Lösungsmitteln.

Die topischen Trägerstoffe können organische oder anorganische Verbindungen sein. Typische pharmazeutische gebrauchte Trägerstoffe sind wäßrige Lösungen, die z.B. Puffersysteme oder isotonische Mischungen von Wasser und wassermischbaren Lösungsmitteln sind, wie z.B. Alkohole oder Arylalkohole, Öle, Polyalkylenglykole, Ethylcellulose, Carboxymethylcellulose, Polyvinylpyrrolidon oder Isopropylmyristat. Geeignete Puffersubstanzen sind z.B. Natriumborat, Natriumphosphat, Natriumacetat oder auch Gluconatpuffer. Die topische Anwendungsform kann auch nicht toxische Hilfsstoffe enthalten wie z.B. emulgierende Konservierungsstoffe, Vernetzer, wie z.B. Polyethylenglykole und antibakterielle Verbindungen.

### Beispiel 1

### Bestimmung der Inhibitor-Konzentration durch Thrombin-Titration

10 bis 100 µl der Inhibitor-Lösung mit einem vorher bestimmten Proteingehalt werden mit 200 µl Natriumhydrogencarbonat-Lösung (pH= 7,0, 05 M) versetzt. Man addiert 0,1 ml Fibrinogen-Lösung (0,5 bis 1 %) oder verdünntes Citrat-Plasma; in regelmäßigem Abstand, unter Rühren, bei Zimmertemperatur, wird ein aliquoter Teil (50 - 100 µl) der Thrombin-Lösung zugefügt (ca. 100 NIH Einheiten pro ml). Als Umschlagpunkt kann beim halbquantitativen Arbeiten das Gerinnen der Flüssigkeit innerhalb des gewählten Zeitabstandes dienen, oder, für quantitative Bestimmungen, die Turbimetrie-Messung bei 546 nm.

### Beispiel 2

Es werden freilebende Blutegel (keine Zuchttiere) der Art Hirudo medicinalis verwendet, die in Deutschland gesammelt worden sind.
Ca. 150 - 200 g gefrorene Blutegelvorderteile werden in einem Mixer mit 2 l eiskalter 0,09 % Natriumchlorid-Lösung und 10 ml Octanol innerhalb von 3 Minuten homogenisiert.
Nach 30 minütiger Zentrifugation bei 0°C und 10 000 Upm wird der Überstand mittels Filtration über 2 Lagen Gaze weitergeklärt und anschließend unter Rühren innerhalb von 15 Minuten auf 80°C erhitzt. Die entstandene Fällung wird durch Filtration über 4 Lagen Gase getrennt. Das Filtrat wird durch Rühren in einem Eisbad schnell auf 4°C heruntergekühlt und in 7,5 l vorgekühltes Aceton (-20°C) gegeben. Es entsteht erneut eine Fällung, die nach 5 Minuten auf einer Glasfilternutsche abfiltriert und mit 1 l kaltem Aceton (-20°C) nachgewaschen wird. Nach Trocknung im Vakuum entsteht 520 mg leicht gelbliches Pulver mit einem Proteingehalt von 62 % (bestimmt nach der Methode von Lowry). Die Antithrombinaktivität beträgt ca. 400 Einheiten pro mg.

### Beispiel 3

520 mg Pulver gemäß Beispiel 1 werden in 75 ml Wasser gelöst, dann mit 5 N Ammoniak auf pH 8,0 eingestellt und 1 Stunde bei 0 - 4°C gerührt. Der unlösliche Anteil wird innerhalb von 30 Minuten mit einer Becher-Zentrifuge bei 5000 Upm abgeschleudert. Nach Einstellung des Proteingehaltes durch Wasserzugabe auf 25 mg/ml (Lowry), wird die Lösung mit 35 ml gesättigter Ammonium sulfat-Lösung versetzt und 1 Stunde bei 4°C gerührt. Der erste Niederschlag wird schnell durch Zentrifugation (5000 Upm/30 Minuten) abgetrennt. Man löst noch ca. 26 g Ammoniumsulfat dazu und stellt den pH-Wert mit Eisessig auf pH 4 ein. Nach 5 Stunden Stehen wird die gesamte Suspension zentrifugiert und der erhaltene Feuchtniederschlag wie folgt weiterverarbeitet.

### Beispiel 4

Der gemäß Beispiel 3 erhaltenen Feuchtniederschlag wird in 200 ml 0,1 M. Ammoniumhydrogencarbonat-Lösung vom pH 8 gelöst und in einer 250 ml Amicon^{R}-Zelle mit einer 5PM 10-Flachmembran (Ausschlußgrenze 10 000 Dalton) ultrafiltriert. Dabei wird die Lösung auf ca. 40 ml eingeengt, wobei gegen Ende zweimal 150 ml 0,1 Ammoniumhydrogencarbonat-Lösung vom pH 8,0 nachgefüllt werden. Gefriertrocknung des Rückstandes ergibt ca. 350 mg Material mit einen Proteingehalt von 89 %.

Man füllt unter Schütteln (Vibromischer) eine C-Säule (Merck) mit Kieselgel 50 µm 100 Å (Grace). 500 mg Extrakt aus Beispiel 4 werden gelöst in 5 ml eines Gemisches aus Chloroform, Methanol, Eisessig, Wasser und Triethylamin im Volumenverhältnis 1200 : 1200 : 4 : 400 : 12 mit 0,2 % Ammoniumacetat auf die Säule aufgebracht. Man eluiert die Säule mit dem gleichen Gemisch in einer Geschwindigkeit von ca. 4 ml/min und fraktioniert in 5 - 10 ml-Portionen. Nach Trocknung des Eluats werden die inhibitorhaltigen Fraktionen in Natriumhydrogencarbonat (pH= 7,0, 0,5 M) gelöst und gesammelt. Die Ausbeute beträgt ca. 50 mg die Aktivität 4000 ATU/mg.

### Beispiel 6

20 mg Inhibitor gemäß Beispiel 3 werden in 200 µl Wasser von pH 2,16 (eingestellt mit Trifluoressigsäure + 5 % Acetonitril) gelöst und auf eine mit Octadecylsilan-Kieselgel (5 µm) gefüllte Stahl-Säule eingespritzt (Shandon^{R}ODS). Die Säule wird durch einen Gradienten von maximal 2 %/Minute zwischen dem Startpuffer (Wasser-pH= 2,16 + 5 % Acetonitril) und dem Endpuffer (Acetonitril/Wasser pH= 2,16 80/20) eluiert. Die Fraktionen werden einzeln gesammelt. Nach Trocknung hat der erfindungsgemäß Inhibitor der Formel II (R= H bzw. SO₃H) eine spezifische Aktivität, die der Stöchiometrie eines 1 : 1-Kompexes mit Thrombin entspricht.

## Patentansprüche

1. Polypeptid der Formel I 
1 2 3 4 5 6 7 8 9 (I)
H—Y—Tyr—Thr—Asp—Cys—Thr—Glu—Ser—Gly—

10 11 12 13 14 15 16 17 18 19
—Gln—Asn—Leu—Cys—Leu—Cys—Glu—Gly—Ser—Asn—

20 21 22 23 24 25 26 27 28 29
—Val—Cys—Gly—Gln—Gly—Asn—Lys—Cys—Ile—Leu—

30 31 32 33 34 35 36 37 38 39
—Gly—Ser—Asp—Gly—Glu—Lys—Asn—Gln—Cys—Val—

40 41 42 43 44 45 46 47 48 49
—Thr—Gly—Glu—Gly—Thr—Pro—Lys—Pro—Gln—Ser—

50 51 52 53 54 55 56 57 58 59
—His—Asn—Asp—Gly—Asp—Phe—Glu—Glu—Ile—Pro—

60 61 62 63 64
—Glu—Glu—Tyr(R)—Leu—Gln—OH
 in welcher
R phenolischer Wasserstoff oder SO₃H bedeutet,
Y Val, Ile, Thr, Leu oder Phe bedeutet und in der jeweils 2 der 6 Cys-Reste in den Positionen 5, 13, 21, 27 oder 38 über Disulfid-Brücken verknüpft sind,
sowie dessen physiologisch verträglichen Salze.

2. Polypeptid nach Anspruch 1, in welcher Y Thr bedeutet.

3. Aus Blutegeln gewonnenes Polypeptid mit der Aminosäurezusammensetzung (Methode von Moore und Stein): 9 Asparaginsäure, 5 Threonin, 4 Serin, 13 Glutaminsäure, 3 Prolin, 9 Glycin, 2 Valin, 6 Cystein, 2 Isoleucin, 4 Leucin, 2 Thyrosin, 1 Phenylalanin, 3 Lysin, 1 Histidin, worin die OH-Gruppe eines der Tyrosinreste mit Schwefelsäure verestert sein kann.

4. Verfahren zur Gewinnung eines gereinigten Polypeptids gemäß Anspruch 1, 2 oder 3, dadurch gekennzeichnet, daß man das Polypeptid aus Würmern der Ordnung Gnathobdellida mit Hilfe einer Kombination von Extraktionsmethoden, Fällungsmethoden, Membranfiltration und/oder chromatographischen Verfahren isoliert und das erhaltene Peptid gegebenenfalls in seine physiologisch verträglichen Salze überführt.

5. Verfahren gemäß Anspruch 4, dadurch gekennzeichnet, daß die Isolierung aus Würmern der Gattung Hirudo erfolgt.

6. Polypeptid gemäß einem der Ansprüche 1, 2 oder 3 zur Anwendung als Heilmittel.

7. Polypeptid gemäß einem der Ansprüche 1, 2 oder 3 zur Anwendung als Antikoagulans.

8. Mittel enthaltend ein Polypeptid gemäß einem der Ansprüche 1 bis 3 und einen pharmazeutisch unbedenklichen Träger.

## Claims

1. A polypeptide of the formula I 
1 2 3 4 5 6 7 8 9 (I)
H—Y—Tyr—Thr—Asp—Cys—Thr—Glu—Ser—Gly—

10 11 12 13 14 15 16 17 18 19
—Gln—Asn—Leu—Cys—Leu—Cys—Glu—Gly—Ser—Asn—

20 21 22 23 24 25 26 27 28 29
—Val—Cys—Gly—Gln—Gly—Asn—Lys—Cys—Ile—Leu—

30 31 32 33 34 35 36 37 38 39
—Gly—Ser—Asp—Gly—Glu—Lys—Asn—Gln—Cys—Val—

40 41 42 43 44 45 46 47 48 49
—Thr—Gly—Glu—Gly—Thr—Pro—Lys—Pro—Gln—Ser—

50 51 52 53 54 55 56 57 58 59
—His—Asn—Asp—Gly—Asp—Phe—Glu—Glu—Ile—Pro—

60 61 62 63 64
—Glu—Glu—Tyr(R)—Leu—Gln—OH
 in which
R denotes phenolic hydrogen or SO₃H,
Y denotes Val, Ile, Thr, Leu or Phe, and in which in each case 2 of the 6 Cys residues at positions 5, 13, 21, 27 or 38 are linked via disulfide bridges,
as well as its physiologically tolerated salts.

2. A polypeptide as claimed in claim 1, in which Y denotes Thr.

3. A polypeptide, obtained from leeches, having the amino acid composition (method of Moore and Stein): 9 aspartic acid, 5 threonine, 4 serine, 13 glutamic acid, 3 proline, 9 glycine, 2 valine, 6 cystein, 2 isoleucine, 4 leucine, 2 tyrosine, 1 phenylalanine, 3 lysine and 1 histidine, in which the OH group of one of the tyrosine residues can be esterified with sulfuric acid.

4. A process for obtaining a purified polypeptide as claimed in claim 1, 2 or 3, which comprises the polypeptide being isolated from worms of the order Gnathobdellida using a combination of extraction methods, precipitation methods, membrane filtration and/or chromatographic processes, and the resulting peptide being, where appropriate, converted into its physiologically tolerated salts.

5. The process as claimed in claim 4, wherein the isolation is from worms of the genus Hirudo.

6. A polypeptide as claimed in any of claims 1, 2 or 3 for use as medicament.

7. A polypeptide as claimed in any of claims 1, 2 or 3 for use as anticoagulant.

8. An agent containing a polypeptide as claimed in any of claims 1 to 3 and a pharmaceutically acceptable vehicle.

## Revendications

1. Polypeptide de formule I 
1 2 3 4 5 6 7 8 9 (I)
H—Y—Tyr—Thr—Asp—Cys—Thr—Glu—Ser—Gly—

10 11 12 13 14 15 16 17 18 19
—Gln—Asn—Leu—Cys—Leu—Cys—Glu—Gly—Ser—Asn—

20 21 22 23 24 25 26 27 28 29
—Val—Cys—Gly—Gln—Gly—Asn—Lys—Cys—Ile—Leu—

30 31 32 33 34 35 36 37 38 39
—Gly—Ser—Asp—Gly—Glu—Lys—Asn—Gln—Cys—Val—

40 41 42 43 44 45 46 47 48 49
—Thr—Gly—Glu—Gly—Thr—Pro—Lys—Pro—Gln—Ser—

50 51 52 53 54 55 56 57 58 59
—His—Asn—Asp—Gly—Asp—Phe—Glu—Glu—Ile—Pro—

60 61 62 63 64
—Glu—Glu—Tyr(R)—Leu—Gln—OH
 dans laquelle
R représente un atome d'hydrogène phénolique ou SO₃H,
Y représente Val, Ile, Thr, Leu ou Phe, et dans laquelle chaque fois 2 des 6 résidus Cys en positions 5, 13, 15, 21, 27 ou 38 sont reliés par des ponts disulfure,
ainsi que ses sels physiologiquement compatibles.

2. Polypeptide selon la revendication 1, dans lequel Y est Thr.

3. Polypeptide, obtenu à partir de sangsues, dont la composition en acides aminés (méthode de Moore et Stein) est la suivante: acide aspartique 9, thréonine 5, sérine 4, acide glutamique 13, proline 3, glycine 9, valine 2, cystéine 6, isoleucine 2, leucine 4, tyrosine 2, phénylalanine 1, lysine 3 et histidine 1, dans lequel le groupe OH d'un des résidus tyrosine peut être estérifié par l'acide sulfurique.

4. Procédé d'obtention d'un polypeptide purifié selon une quelconque des revendications 1 à 3, caractérisé en ce qu'on isole le polypeptide à partir de vers de l'ordre des gnathobdellidés, à l'aide d'une combinaison de procédés d'extraction, de précipitation, de filtration membranaire et/ou de chromatographie, et qu'on transforme éventuellement le peptide obtenu en ses sels physiologiquement compatibles.

5. Procédé selon la revendication 4, caractérisé en ce que l'isolement est effectué a partir de vers du genre Hirudo.

6. Polypeptide purifié selon une quelconque des revendications 1 à 3, pour son utilisation comme médicament.

7. Polypeptide purifié selon une quelconque des revendications 1 à 3, pour son utilisation comme anticoagulant.

8. Médicament contenant un polypeptide selon une quelconque des revendications 1 à 3 ainsi qu'un véhicule pharmaceutiquement acceptable.
